# EUROPEAN PATENT APPLICATION

(11) **EP 4 173 642 A1**
(43) Date of publication of application: **03.05.2023**
(21) Application number: 21833450.6
(22) Date of filing: 25.06.2021
(51) Int. Cl.: A61L 2/10, A61N 5/06

(54) **SELF-DISINFECTING TOUCH-SENSING SURFACE**

(30) Priority: 30.06.2020 BR 102020013480
(71) Applicant: Mendes, Rafael Viana, 29065-420 Vitória - ES (BR)
(72) Inventor: Mendes, Rafael Viana, 29065-420 Vitória - ES (BR)
(74) Representative: Girardi, Giovanna Paola
(86) International application number: PCT/BR2021/050282
(87) International publication number: WO 2022/000061

(57) **Abstract**

The present invention deals with a touch detection surface with application in the area of electrical and/or electronic devices encompassing touch screens and similar devices, which have at least one UV radiation emitter. This invention, in turn, is capable of decontaminating the area used by the device operator both after the operator's contact with the surface, and after the release of data, as well as in a previously programmed manner.

## Description

1. The present invention deals with a touch detection surface with application in the field of electrical and/or electronic devices with at least one UV radiation emitter. The invention combines the germicidal or disinfectant effectiveness of UV electromagnetic radiation and the protection of said radiation in a local region to the contact surface of the switch, so as to sanitize the contact area of the touch detection surface of interest without harmful exposure or from humans or animals to UV radiation.

2. Touch detection surfaces are elements which, when touching one or more points on their surface, release and/or interrupt and/or intensify and/or reduce the passage of electrical current in a given circuit. Touch sensing surfaces include touch screens, multi-touch screens, resistive touch screen, projected capacitive touch screens, integrated touch screen, among other devices that allow the same functionality.

3. Currently, touch detection surfaces have their operator contact surfaces constructed of materials with a high possibility of proliferation of viruses and bacteria, thus allowing the risk of human contamination.

4 It is well known that, in environments with a high flow of people in which the activation of electrical and/or electronic circuits takes place through touch detection surfaces, the risk of contamination and consequent propagation of viruses and bacteria among users of these devices.

5 In order to mitigate or solve this risk of mass contamination, the present invention arose, since, through this developed tool, it became possible to autonomously decontaminate the surfaces which, through touch detection, trigger up electrical and/or electronic circuits.

6. Ultraviolet light has been shown to be effective in eliminating or inactivating most harmful microbial substances. Ultraviolet light is normally divided into three subcategories according to its wavelength of light or electromagnetic radiation which comprises the spectrum of said light.

Petition 870200148181, dated Nov 24/, 2020, p. 3/5

7. These categories are commonly known as UV A, UV B or UV C. UV A is generally composed of electromagnetic radiation wavelengths mainly in the range of 320 nm to 400 nm, while UV B is generally composed of electromagnetic radiation wavelengths mostly in the 280nm to 320nm range and UV C is generally composed of wavelengths less than 280nm.

8. Of these three types of ultraviolet electromagnetic radiation, UV C is generally considered to have the greatest effectiveness in killing or inactivating germs. For example, germicidal ultraviolet radiation of a wavelength of 254 nm is generally accepted to begin killing or passivating microbes with an exposure of about 2,000 µW-s / cm² with complete elimination or inactivation occurring by exposures in the range of about 12,000 µW-s / cm².

9. For this, it is necessary at least one radiation source, which radiates UV-C rays on the contact surface, which may be located on the inside or outside of the device, being energized by the electrical circuit itself or by an additional electrical circuit, depending on the application and depending on the model of the touch detection surfaces.

10. The surface to be decontaminated must receive more than 2000 µW-s / cm² per decontamination cycle.

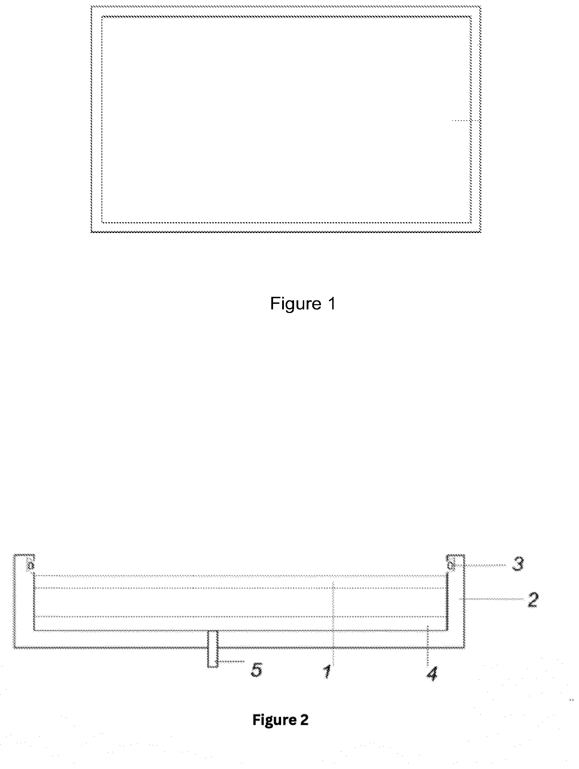

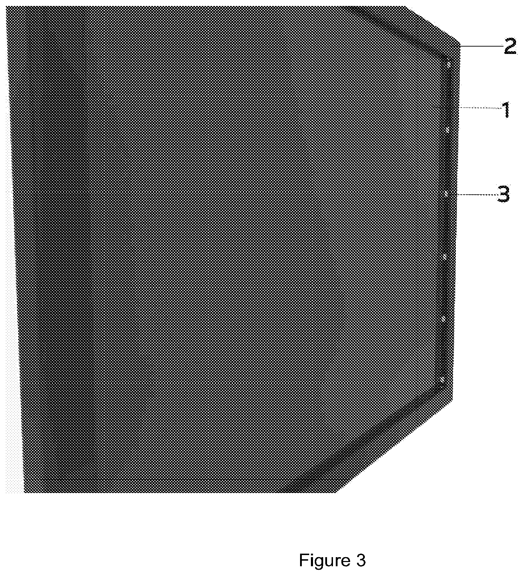

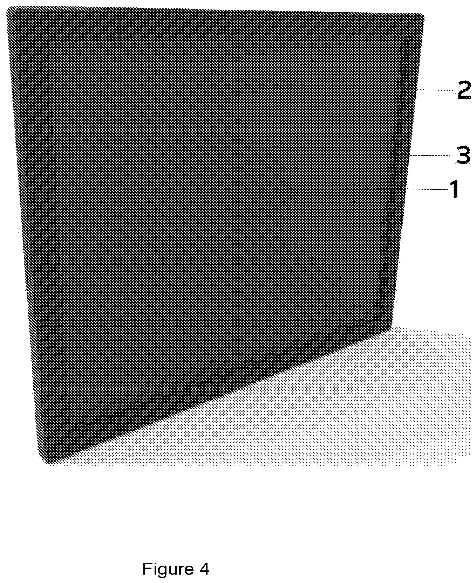

11. The invention may be better understood through the following illustrative description, of a model in which the invention is applied, in line with the attached figures, where: figure 1 represents a cross-sectional view of the touch detection surface and Figure 2 represents a front view, in section, of the touch detection surface.

12. The decontamination of the touch-sensitive screen happens due to UV-C radiation, which is considered effective against microorganisms harmful to human beings, however, for the invention to be effective, it is essential that the UV radiation reaches every surface that comes in contact with the operator.

13. The radiation source can be located on the inside of the covering body and/or on the outside, and can also be located behind protective layers, such as the case of impact glass and/or between layers of translucent or transparent materials, since that radiation can effectively decontaminate the contact area used by the operator.

14. With reference to these figures, it can be seen that the covering body (2) is equipped with a circuit board (4) that receives electrical current and transfers data through the wires (5), being connected to a touch-sensitive surface (1 ) which is irradiated by LED's emitting UV-C radiation (3).

15. There is an inclination of about 30 degrees in the covering body (2) on which the radiation emitters are fixed. This inclination allows the radiation to be concentrated on the surface of the touch screen (1).

16. The LEDs (3) can be supplied electrically via the circuit board (4) or via a parallel electrical circuit.

17. Radiation emitters can operate uninterruptedly or only when required by device or system programming. Thus, decontamination can occur either from the physical touch on the sensitive surface, that is, with energy release, or from the release of data by the electrical and/or electronic circuit and by means of previously defined programming.

18. The radiation from the LED can be directed using optical fibers, as long as its final power is able to effectively decontaminate the entire proposed surface.

## Claims

1. Touch detection surface with the ability to recognize the presence of contact points having at least one UV radiation emitter (3) with a wavelength between 100nm and 380nm capable of decontaminating the external part of the contact area (1) with the device operator, having an electrical circuit that can be programmed the best time when the radiation emitter will be turned on and off; **characterized in that** it can be equipped with a reflector (2) to reflect the radiation emitted by at least one and/or more radiation emitters in order to concentrate the radiation in a certain area.

2. Touch detection surface with at least one UV radiation emitter according to claim 1, **characterized in that** it has at least one UV radiation emitter (3) located outside the device, which can be located inside one or more places.

3. Touch detection surface with at least one UV radiation emitter according to claim 1, **characterized in that** it can have the UV radiation directed by optical fiber (5) to the region to be decontaminated.

4. Touch detection surface with at least one UV radiation emitter according to claim 1, **characterized in that** there is a film on the surface of the screen (1) to protect the components from UV radiation.

5. Touch detection surface with at least one UV radiation emitter according to claim 1, **characterized in that** it comprises touch-sensitive and multi-touch screens.

6. Touch detection surface with at least one UV radiation emitter according to claim 1, **characterized in that** it can have at least one lighting LED (3) indicating that the detection surface is decontaminated.

7. Touch detection surface with at least one UV radiation emitter according to claim 1, **characterized in that** they can comprise touch screens, multi-touch screens, resistive touch screen, projected capacitive touch screens, integrated touch screen.
